# EUROPEAN PATENT SPECIFICATION

(11) **EP 0 829 540 B1**
(45) Date of publication and mention of the grant of the patent: **21.11.2001**
(21) Application number: 97307081.6
(22) Date of filing: 12.09.1997
(51) Int. Cl.: C12N 15/79, C12N 15/19, A61K 39/002, A61K 39/005, A61K 39/008, C07K 14/535

(54) **Macrophage-infecting parasites expressing a granulocyte macrophage colony stimulating factor**
Makrophagen infizierende Parasiten, die ein Leukozyten- und Makrophagen-stimulierendes Hormon exprimieren
Parasites infectant les macrophages, qui expriment un facteur stimulant la croissance de colonies de macrophages et de granulocytes

(30) Priority: 13.09.1996 US 713768
(43) Date of publication of application: 18.03.1998
(73) Proprietor: Université Laval, Sainte-Foy, Quebec G1V 4G2 (CA)
(72) Inventor: Papadopoulou, Barbara, Quebec G1S 2Y2 (CA); Ouellette, Marc, Quebec G1S 2Y2 (CA); Olivier, Martin, Quebec G1R 2X8 (CA)
(74) Representative: Smart, Peter John

(56) References cited:
- AL-ZAMEL, FATEN ET AL: "Enhancement of leishmanicidal activity of human macrophages against Leishmania major and Leishmania donovani infection using recombinant human granulocyte macrophage colony stimulating factor" ZENTRALBL. BAKTERIOL. (1996), 285(1), 92-105 CODEN: ZEBAE8;ISSN: 0934-8840, September 1996, XP002050656
- ELIZABETH OLIVARES FONTT ET AL: "Granulocyte-macrophage colony-stimulating factor: involvement in control of Trypanosoma cruzi infection in mice" INFECT. IMMUN. (1996), 64(8), 3429-3434 CODEN: INFIBR;ISSN: 0019-9567, August 1996, XP002050657
- MODABBER, F.: "Vaccines against leishmaniasis" ANN. TROP. MED. PARASITOL. (1995), 89(SUPPL. 1), 83-88 CODEN: ATMPA2;ISSN: 0003-4983, XP002050658
- BADARO R ET AL: "Recombinant human granulocyte-macrophage colony-stimulating factor reverses neutropenia and reduces secondary infections in visceral leishmaniasis" J. INFECT. DIS, vol. 170, no. 2, 1994, pages 413-8, XP002050659
- TOBIN, JAMES F. ET AL: "A sequence insertion targeting vector for Leishmania enriettii" J. BIOL. CHEM. (1992), 267(7), 4752-8 CODEN: JBCHA3;ISSN: 0021-9258, XP002050660
- HO J H ET AL: "Granulocyte-macrophage and macrophage colony-stimulating factors activate intramacrophage killing of Leishmania mexicana amazonensis" J. INFECT. DIS., vol. 162, no. 1, 1990, pages 224-30, XP002050661
- Dorland's Illustrated, Medical Dictionary, Twenty-sixth Edition, W.B. Saunders Company, Philadelphia, 1981, page 968

## Description

### FIELD OF INVENTION

The present invention relates to the field of molecular immunobiology and in particular to immunogenic preparations including vaccines comprising attenuated parasites.

### BACKGROUND OF INVENTION

Parasite infection is responsible for a number of human and animal diseases.

Parasitic protozoa of the order Kinetoplastidae are the causative agents of several tropical diseases including sleeping sickness by *Trypanosoma brucei,* Chagas by *Trypanosoma cruzi,* visceral (kala-azar) and cutaneous (oriental sore) Leishmaniasis by *Leishmania donovani* and *Leishmania major* respectively. In particular *Leishmania* protozoans are the causative agents of human leishmaniasis, which includes a spectrum of diseases ranging from self-healing skin ulcers to fatal visceral infections. Human leishmaniasis is caused by at least thirteen different species and subspecies of parasites of the genus *Leishmania.* Leishmaniasis has been reported from about eighty countries and probably some 400,000 new cases occur each year. Recently the World Health Organization has reported 12 million people to be infected (ref. 1. Throughout this application various references are referred to in parenthesis to more fully describe the state of the art to which this invention pertains. Full bibliographic information for each citation is found at the end of the specification, immediately preceding the claims. The disclosure of these references are hereby incorporated by reference into the present disclosure. A listing of the references appears at the end of the disclosure).

Untreated visceral leishmaniasis is usually fatal and mucocutaneous leishmaniasis produces mutilation by destruction of the naso-oropharyngeal cavity and, in some cases, death.

In addition a major health problem has been created in areas of high infection when blood is collected for transfusion purposes. Since blood is a carrier of the parasites, blood from an infected individual may be unknowingly transferred to a healthy individual.

The *Leishmania* protozoans exist as extracellular flagellated promastigotes in the alimentary tract of the sandfly in the free-living state, and are transmitted to the mammalian host through the bite of the insect vector. Once introduced, the promastigotes are taken up by macrophages, rapidly differentiate into non-flagellated amastigotes and start to multiply within the phagolysosomal compartment. As the infected cells rupture, amastigotes subsequently infect other macrophages giving rise to the various symptoms associated with leishmaniasis (refs. 1 and 2).

Leishmaniasis is, therefore, a serious disease and various types of vaccines against the disease have been developed, including live parasites; frozen promastigotes from culture; sonicated promastigotes; gamma-irradiated live promastigotes; and formalin-killed promastigotes treated with glucan (reviewed in, for example ref. 3). However, none of these approaches have provided efficacious vaccines.

Healing and progression of leishmaniasis are linked to the dissimilar expansion of functionally distinct CD4+ lymphocyte responses separated on the basis of their cytokine potential (ref. 4). T helper type 1 (Th1) subset produces interferon (IFN)-,γ and interleukin (IL)-2 and leads to resistance to *Leishmania* infection, whereas Th2 cells producing IL-4, IL-5 and IL-10 confer susceptibility (ref. 5). In mammalian hosts, *Leishmania* reside exclusively within mononuclear phagocytes, macrophages and monocytes. Cytokines can modulate macrophage differentiation by causing selective changes in macrophage gene expression, leading to alterations on macrophage functions (ref. 6). Macrophages, pre-incubated *in vitro* with cytokines prior to infection with *Leishmania,* acquire the capacity to kill the intracellular parasites (refs. 7 to 11). Furthermore, cytokines such as IFN-γ, TNF-α, IL-12 and GM-CSF have been used in anti-leishmanial therapy in experimental models (refs. 12 to 17). Expression of the IFN-γ gene has been performed in *L. major* trypanosomatids. When nude mice were infected with the IFN-γ expressing transfectant, the progression of the disease was considerably slower (ref. 17). The progression of the disease was not retarded in susceptible BALB/c mice however.

GM-CSF is a cytokine with multipontential hematopoietic function, stimulating the formation of granulocytes, macrophages, and eosinophils (ref. 18). It activates macrophage tumoricidal activity (ref. 19), increases macrophage killing of *Trypanosoma cruzi* (ref. 20) and enhances *in vitro* killing of *L. donovani* within macrophages (ref. 8).

Differences on the effect of this cytokine have been reported between experimental visceral and cutaneous infections (ref. 27). Although in the *L. donovani* model, GM-CSF demonstrates a clear-cut leishmanicidal activity *in vitro* and *in vivo* (refs. 28), the results obtained with *L. major* are conflicting suggesting that GM-CSF may play a positive (refs. 29, 10, 6), neutral (refs. 30, 4) or negative (ref. 31) host defense role.

Parasitic infection of macrophages and, in particular, *Leishmania* infection may lead to serious disease. It would be advantageous to provide attenuated strains of *Leishmania* and methods of production thereof, for use as antigens in immunogenic preparations, including vaccines, and the generation of diagnostic reagents.

### SUMMARY OF THE INVENTION

The present invention is directed towards novel strains of *Leishmania* and other macrophage-infecting parasites, particularly attenuated strains. The novel macrophage-infecting parasites provided herein are useful for the preparation of immunogenic preparations including formulations for the treatment of hosts infected by *Leishmania* and vaccines against disease caused by infection by a virulent *Leishmania* strain and as tools for the generation of immunological and diagnostic reagents.

In accordance with one aspect of the present invention, there is provided a macrophage-infecting parasitic protozoan of the order Kinetoplastidae expressing a granulocyte macrophage colony stimulating factor (GM-CSF) gene. The parasite may be a strain of *Leishmania,* including a strain of *Leishmania* selected from the group consisting of *Leishmania donavani, Leishmania braziliensis, Leishmania tarentolae, Leishmania a major, Leishmania mexicana, Leishmania tropica and Leishmania aethiopica.*

The parasite may be one which is reduced in the ability of the strain to infect or survive in macrophages and hence is attenuated. Further, at least one gene of the parasite contributing to virulence thereof may be functionally diabled. Additionally, the parasite may be further modified to express at least one additional cytokine which may be macrophage-activating.

The GM-CSF may be of murine origin or human origin. Expression of the GM-CSF gene from the parasite may be achieved by providing a plasmid into which the GM-CSF gene is inserted downstream of a promoter. For *Leishmania,* the intergenic region of the α-tubulin gene of *L. enriettii* may be used and the GM-CSF gene may be inserted into a *Leishmania* expression vector, which may be a plasmid.

In a further aspect, the present invention provides an immunogenic composition comprising an attenuated form of the parasites as provided herein.

The immunogenic composition, for the parasite being a strain of *Leishmania* may be formulated for *in vivo* administration to a host, such as a primate, including humans, infected by *Leishmania* to treat such infection.

The immunogenic composition, for the parasite being a strain of *Leishmania,* may be formulated as a vaccine for *in vivo* administration to a host, such as a primate including humans, to confer protection against disease caused by a virulent strain of *Leishmania,* including *Leishmania donovani, Leishmania braziliensis, Leishmania tarentolae, Leishmania major, Leishmania mexicana, Leishmania tropica* and *Leishmania aethiopica.*

In an additional aspect, the invention provides means for performing a method of generating an immune response in a host, such as, a primate including humans, comprising administering thereto an immunoeffective amount of the immunogenic composition, as provided herein.

In yet an additional aspect, there is provided means for performing a method for producing a vaccine for protection against a disease caused by infection by a virulent strain of a macrophage-infecting parasite, including a virulent strain of *Leishmania,* including *Leishmania donovani, Leishmania braziliensis, Leishmania tarentolae,* *Leishmania mexicana, Leishmania tropica* and *Leishmania aethiopica,* and comprising administering the immunogenic composition as provided herein to a test host to determine an amount and frequency of administration thereof to confer protection against disease caused by infection by the *Leishmania* parasite and formulating the immunogenic composition in a form suitable for administration to a treated host, including humans, in accordance with said determined amount and frequency of administration.

Advantages of the present invention include the provision of safe and attenuated strains of *Leishmania* and other macrophage-infecting parasites for the preparation of immunogenic compositions including vaccines and for the generation of immunological and diagnostic reagents.

### BRIEF DESCRIPTION OF THE DRAWINGS

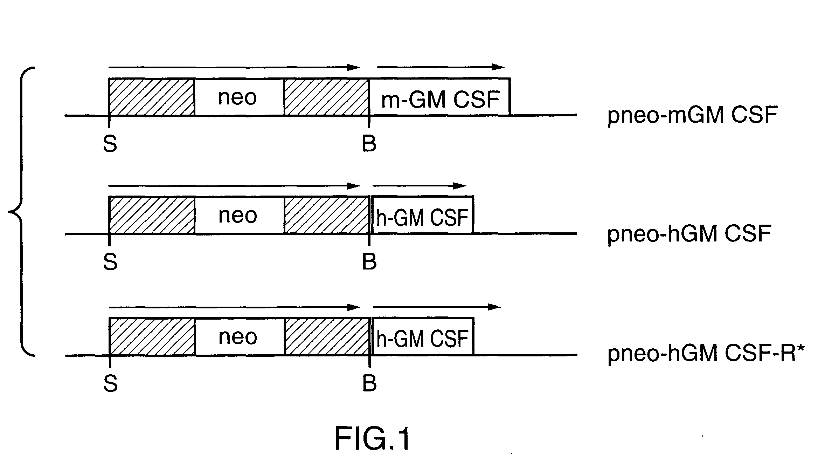

Figure 1 shows the construction of *Leishmania* vectors expressing GM-CSF. Plasmids pneo-mGM CSF and pneo-hGM CSF were made by inserting the murine and human GM-CSF genes, respectively downstream of the intergenic region of the α-tubulin gene of *L. enriettii* into pSP72-αneoα *Leishmania* expression vector as described in the Examples below. The cross-hatch box corresponds to the α-tubulin gene intergenic region (ref. 21). S=*Sma*I, B-*Bnm*HI. Arrows indicate the orientation of transcription of the genes present in the expression vectors.

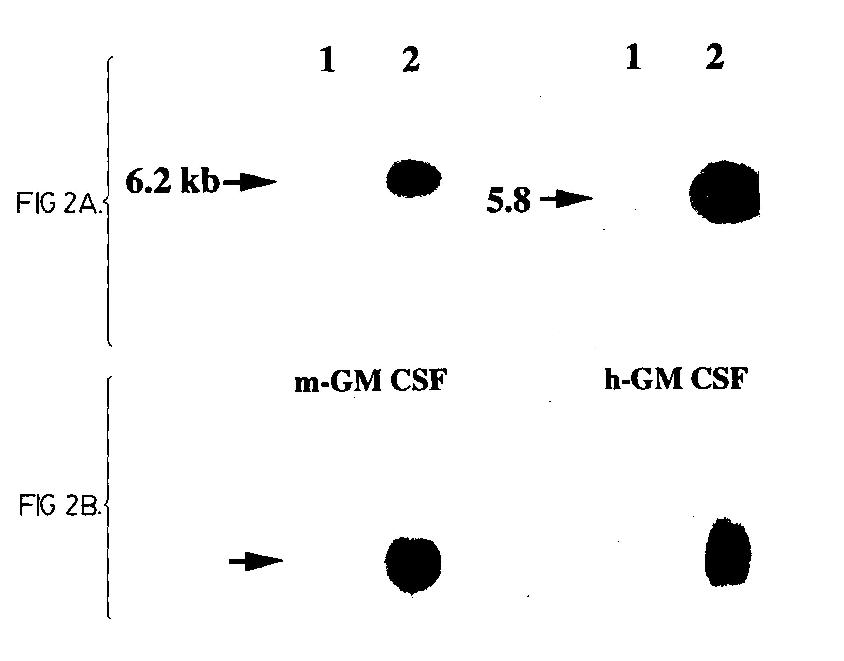

Figure 2 consists of two panels, A and B. Panel A is a southern blot of total genomic DNAs of *L. major* digested with *Bgl*II and hybridized to murine and human GM-CSF specific probes. Lane 1 corresponds to *L. major*-pneo and Lane 2 corresponds to *L. major* with either the murine or the human GM-CSF gene. Panel B shows mRNA expression in the *L. major*-GM CSF expressing cells.

Northern blot of total *Leishmania* RNAs hybridized to the same probes as with panel A above. Lanes are as in A.

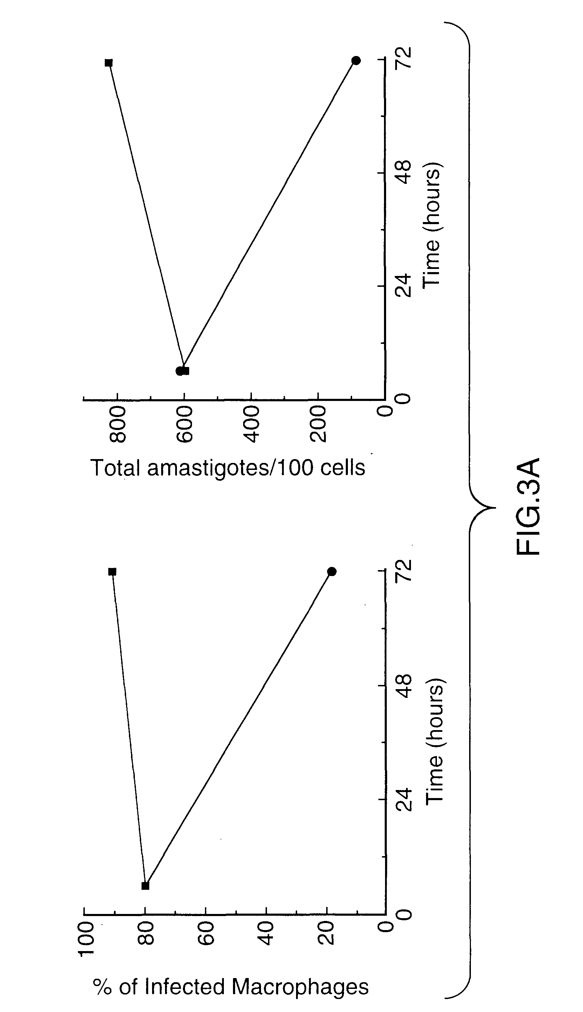

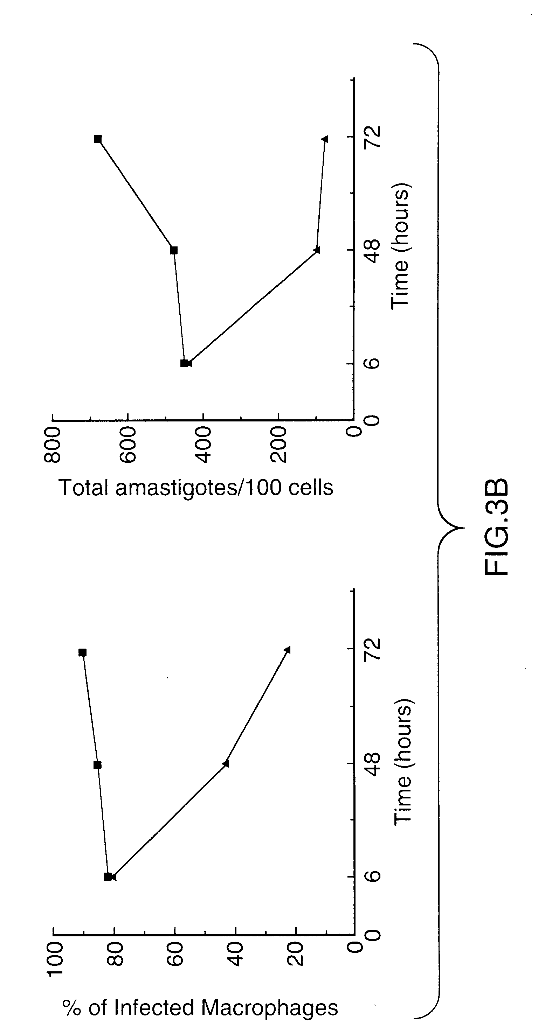

Figure 3, comprising panels A and B, contains graphical representations of intramacrophage killing of *Leishmania donovani* amastigotes expressing the murine and human GM-CSF genes. *L. donovani* expressing either the murine or the human GM-CSF gene were harvested from stationary phase and counted with the Neubauer improved counting chamber. Murine macrophages from J774 cell line and human monocytes differentiated to macrophages (5 x 10⁴ cells/well) were incubated with stationary phase *L. donovani* parasites (20:1, parasite-to-cell ratio) for 6 hours. After this initial incubation, free parasites were washed and fresh media was added to the wells and incubated for 48 and 72 hours. At these fixed time points cell cultures were dried and stained with Diff Quick in order to determine the level of infection. Panel A shows infection of murine macrophages. Panel B shows infection of human macrophages. The left graph of each panel corresponds to the percentage of infected macrophages and the right graph of each panel corresponds to the total number of amastigotes in 100 macrophage cells with time. ■ *L. donovani*-pneo; ● *L. donovani*-mGM CSF; ▲ *L. donovani*-hGM CSF.

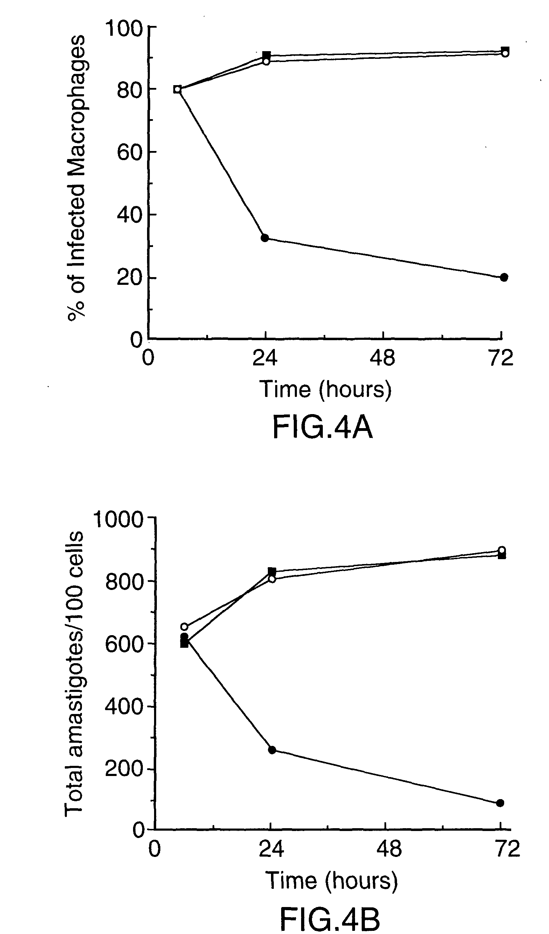

Figure 4, comprising panels A and B, contains graphical representations of intracellular killing of *Leishmania major* amastigotes expressing the human GM-CSF gene. Human monocytes differentiated into macrophages were infected by *L. major* expressing the human GM-CSF gene as described in Fig. 3. Panel A shows the percentage of infected human macrophages with time. Panel B shows the total number of amastigotes in 100 macrophage cells with time. ■ *L. major*-pneo; ● *L. major*-hGM CSF; ○ *L. major-hGM* CSF R* representing the reverse orientation of the GM-CSF gene.

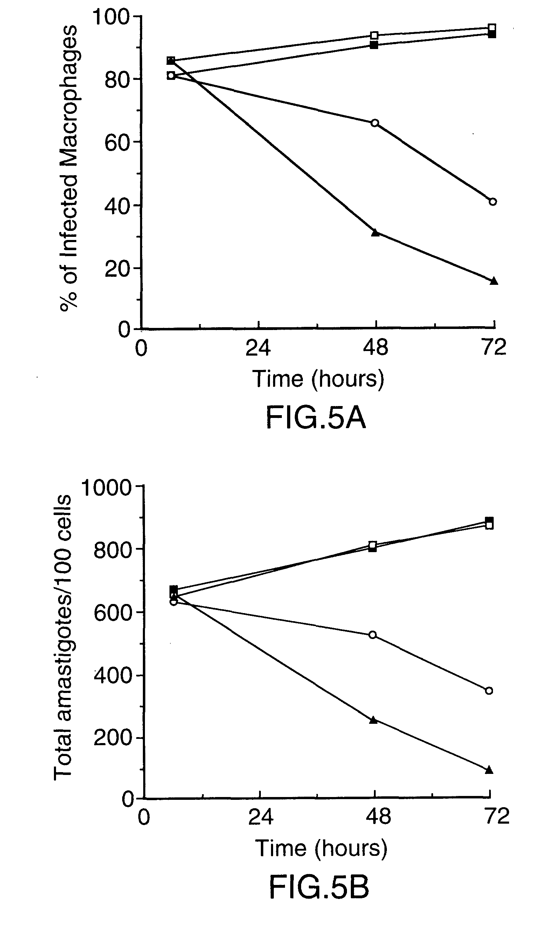

Figure 5, comprising panels A and B, contains graphical representations of intracellular killing of *Leishmania major* amastigotes expressing the murine GM-CSF gene and reversion by an anti-GM CSF antibody. Murine macrophages were infected by *L. major* expressing the murine GM-CSF gene as described in Fig. 3. Panel A shows the percentage of infected murine macrophages with time. Panel B shows the total number of amastigotes in 100 macrophage cells with time. ■ *L. major-*pneo; ▲ *L. major-* mGM CSF; □ *L.* major-pneo infection with macrophages pre-treated with a concentration of an anti-GM CSF antibody capable to neutralize 50% of the GM-CSF activity; O L. major-mGM CSF infection with the same concentration of an anti-GMCSF antibody.

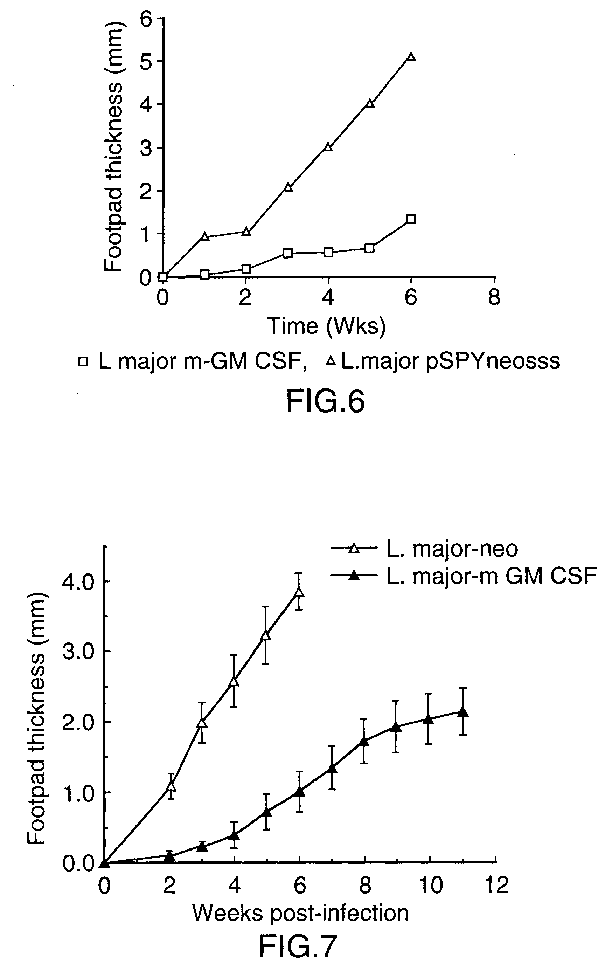

Figure 6 is a graphical representation of the inability of *L. major* expressing GM-CSF to promote infection with BALB/C susceptible mice. 2 x 10² *Leishmania* promastigotes expressing GM-CSF and also wild-type were injected into the footpad of eight mice per group. Infection was monitored by measuring the footpad lesion with a metric caliper over 6 weeks. ■ *L. major* m-GM CSF; ▲ *L. major* pSPYneosss mGM CSF.

Figure 7 is a graphical representation of the effect of GM-CSF in vivo .1 x 10⁷ stationary phase *L. Major* LV39 strain transfected either with pSPYneo (control) or with a mGM-CSF expression vector were used for infecting BALB/c mice. Cutaneous infection in mice was monitored by measuring the thickness in infected footpads with a metric caliper at weekly intervals. The net value for the footpad thickness (in mm) was calculated by substracting the diameter of the uninfected footpad from the one of the infected footpad. The average and standard deviation of the results obtained from six mice per time point are shown. The data shown are representative of one experiment which has been repeated five times with essentially similar results. Δ = *L. Major - neo, ▲ = L. Major - mGM CSF.*

### GENERAL DESCRIPTION OF THE INVENTION

Referring to Figure 1, there is shown vectors for expression of the Granulocyte Macrophage Colony Stimulating Factor (GM-CSF) in *Leishmania.*

To express GM-CSF in *Leishmania* the 800 bp intergenic region of the α-tubulin gene of *L. enriettii* was subcloned upstream of either the murine or human GM-CSF genes. The α-tubulin intergenic region provides the necessary signals for trans-splicing that are required for correct maturation of transcripts in *Leishmania* (ref. 21). The same sequences were used for the expression of the neomycin phosphotransferase gene *(neo)* as part of an *αneo*α cassette cloned in vector pSP72 (Fig. 1). These vectors were transfected by electroporation into *L. major* and *L. donovani* strains and transfectants resistant to G418 were selected on SDM-1% agar plates. Genomic DNAs were isolated from selected clones, digested with *Bgl*II to linearize the GM-CSF expression vectors and analyzed by Southern blot using GM-CSF specific probes either for the murine (m) or the human (h) genes.

5.8 kb and 6.2 kb fragments from the *L. major*-pneo-hGM CSF and pneo-mGM CSF transfectants, respectively were obtained as expected (Fig. 2A). Northern blot analysis of total *L. major* RNA showed that GM-CSF transcripts of the expected size for both the murine and the human genes were produced (Fig. 2B). Identical bands were also obtained for the *L. donovani-*GM CSF transfectants.

The ability of *L. major-* and *L.* donovani-hGM CSF promastigotes to secrete GM-CSF was determined by direct ELISA assay. *Leishmania* cells were grown to stationary phase and the supernatants harvested after centrifugation. As assessed by ELISA, *Leishmania* expressing the hGM-CSF gene were associated with detectable amounts of GM-CSF protein in the media. The concentration of hGM-CSF detected in culture media ranged from 2.8 to 6.2 ng/ml. GM-CSF expressing parasites can be maintained in culture as promastigotes for several months without any obvious effect on growth or morphology. Therefore, GM-CSF expression is not detrimental to *Leishmania.*

Referring to Figure 3, there is shown the intramacrophage killing of *Leishmania donovani* expressing the murine and human GM-CSF genes. Phagocytes were infected with 10⁶ stationary phase *Leishmania*-GM CSF and *Leishmania*-pneo control at a cell ratio of 1:20 for a period of 6 hours. The outcome of the infection *in vitro* was followed at 6, 24, 48, and 72 hours by microscopic examination. The intracellular survival of *Leishmania* amastigotes expressing GM-CSF significantly decreased inside both the murine and the human macrophages. More specifically, *L. donovani* amastigotes expressing either the murine or the human GM-CSF genes were eliminated 7 to 8-fold more rapidly inside macrophages than control cells 72 hours following infection (see Fig. 3A,B). Following the same infection period only 20% of macrophages were infected (Fig. 3A, B). Similar results were observed with *L. major* parasites expressing GM-CSF. Indeed, 72 hours following infection only 18-20% of macrophages were infected compared to 92-95% for the control strain (Figs. 4A and 5A). In addition, a 9-fold decrease was measured in the total amastigote number found inside either human or murine macrophages infected with *L. major-GM-CSF* expressing parasites compared to those infected with the control-neo strain (Figs. 4B and 5B). The decrease in intracellular survival of *Leishmania* amastigotes expressing GM-CSF was a specific effect related to GM-CSF activity. Thus, murine macrophages were infected with *Leishmania* cells expressing either the human or the murine GM-CSF genes. Only parasites expressing the murine GM-CSF gene were eliminated inside macrophages (Fig. 3A). Furthermore, a construct in which the hGM-CSF gene was cloned in the reverse orientation in respect to the α-tubulin intergenic region necessary for expression in *Leishmania* was not capable of inducing parasite elimination in infected macrophages (Figure 4).

Finally, by pre-treating murine macrophages with an anti-mGM CSF polyclonal antibody prior to infection, the anti-leishmanial effect of GM-CSF was significantly decreased (Fig. 5). Indeed, an antibody concentration capable of neutralizing 50% of the GM-CSF activity blocked more than 50% of the intra-macrophage elimination of *L. major*-mGM CSF transfectants compared to the untreated cells (see Fig. 5). Similar results have been obtained using the *L. donovani* parasites.

Thus GM-CSF expression greatly reduces the viability of amastigotes inside murine or human macrophages. GM-CSF induce parasite killing by activating macrophages to enhance H₂O₂ release (refs. 18, 20) or most likely to produce more nitric oxide or to augment IFN-γ and IL-1 production (ref. 10). GM-CSF, as IFN-γ, can increase production of the anti-leishmanial cytokine TNF-α (refs. 12, 13, 32, 33, 15) and some of its biological effects might be amplified through the release of this cytokine.

Referring to Figure 6, there is shown the inability of *L. major* expressing GM-CSF to promote infection of BALB/c susceptible mice. Two series of animals were inoculated into the footpad with either 2 x 10⁷ *L. major-mGM* CSF recombinant parasites (a clone) or with *L.* *major-*pSPYneo control strain. Eight mice were used for each group. The outcome of the infection of these mice was followed by weekly measurement of the footpad lesions. As shown in Fig. 6, after six weeks post-infection, a significant decrease in the development of cutaneous lesion was observed in animals inoculated with the GM-CSF expressing parasites compared to those infected with the neo control strain.

Figure 7 is representative data of a series of experiments in which BALB/c mice were infected with *L*. *major* expressing either the GM-CSF gene or the neo gene as a control. The procedure is the same as that for Figure 6, except that groups of six mice were inoculated with 1 x 10⁷ *L. major* mGM CSF recombinant parasites or with *L. major* as pSPYneo control strain. The net value for footpad thickness was calculated by substracting the diameter of the uninfected footpad from one of the infected footpad. The mice infected with *Leishmania* experessing mGM-CSF were followed for 11 weeks. The average and standard derviation obtained from the six mice per time point are shown in Figure 7.

The mice infected with *Leishmania* expressing GM-CSF showed no inflammation or lesions at the level of the footpad for the first 4 to 5 weeks post-infection compared to the control mice where lesions were very large after the same time period and the mice needed to be sacrified. From the fifth week, inflammation at the level of the footpads infected with GM-CSF expressing parasites started to be visable and slowly increased with time over the three-months of observation. The majority of the mice did not develop important lesions such as those seen with the control parasites.

The *Leishmania*-GM-CSF expressing parasites provided herein provide means to create favorable immune responses in a host. Parasites expressing cytokines provide effective vaccines for leishmaniasis by mediating their own destruction while establishing protective Th1-mediated immunity.

### EXAMPLES

The above disclosure generally describes the present invention. A more complete understanding can be obtained by reference to the following specific Examples. These Examples are described solely for purposes of illustration and are not intended to limit the scope of the invention. Changes in form and substitution of equivalents are contemplated as circumstances may suggest or render expedient. Although specific terms have been employed herein, such terms are intended in a descriptive sense and not for purposes of limitations.

Methods of molecular genetics, protein biochemistry and immunology used but not explicitly described in the disclosure and these Examples are amply reported in the scientific literature and are well within the ability of those skilled in the art.

### Example 1

This Example describes techniques of recombinant DNA.

*Leishmania* vector pneo-mGM CSF expressing the murine-GM-CSF gene was made as follows. A 1.2 kb *Kpn*I-*Kpn*I fragment isolated from plasmid pXMT2 containing the mGM-CSF gene and flanking sequences was filled in with T4 DNA polymerase (New England Biolabs) to create blunt ends and subcloned into the *Bam*HI site of vector pSP72-αneoα filled in with Klenow DNA polymerase (New England Biolabs) to yield pneo-mGM CSF (Figure 1). Vector pSP72-αneoα was generated by subcloning the 2.6 kb *Bam*HI*-Sma*I neo-cassette from pGEM3-neo (ref. 23) containing the *neo* gene flanked by the intergenic regions of the α-tubulin gene of *L. enriettii* (ref. 21) into the *Bam*HI-*Sma*I of pSP72 (Promega). The *Leishmania* construct to express the human GM-CSF gene was made by subcloning an 800 bp *Xho*I-*Xho*I fragment from vector pXMT1 containing the hGM-CSF gene and flanking sequences into the *Sal*I site of pSP72-αneoα to yield pneo-hGM CSF (Figure 1). Construct pneo-hGM CSF R* (Figure 1) corresponds to the reverse orientation of the human GM-CSF gene with respect to the αneoα cassette in such way that specific gene expression cannot occur.

Total genomic DNA from *Leishmania* was prepared as described (ref. 26), digested with *Bgl*II resolved on 0.7% agarose gels and transferred to nylon membranes (Hybond-N, Amersham). Total RNAs from *L. major*-pneo and GM-CSF transfectants were prepared using Trizol (Gibco BRL). Southern and Northern blots, hybridizations and washings were done following standard procedures and the results are shown in Figure 2. The GM-CSF probes used correspond to a 1.2 kb *Kpn*I-*Kpn*I fragment for the mGM-CSF gene and a 800 bp *Xho*I-*Xho*I fragment for the hGM-CSF gene.

### Example 2

This Example describes the culturing and transfection of *Leishmania.*

*Leishmania major* MHOM/IL/67/JERICHO II; and *Leishmania donovani* MHOM/IN/80/DD8 are WRAIR/WHO reference strains obtained from the ATCC. All strains were grown in SDM-79 medium (ref. 22) supplemented with 10% fetal bovine serum (FBS) (Multicell, Wisent Inc.) and 5 µg/ml of hemin. Approximately 15 µg of pneo-GM CSF vector DNA was used to transfect *L. donovani* and *L. major* cells by electroporation as described (refs. 23, 24). Transfectants were selected with 40 µg/ml of G-418 (Geneticin, Gibco-BRL). *Leishmania*-GM CSF and control *Leishmania*-pneo transfectants were seeded at low concentration (3 x 10⁴ cells per culture dish) in 5 ml of SDM-79 medium. After 72 hours the cell density was obtained by measuring the absorbance at 600 nm using an automated microplate reader (Reader 510 from Organon Tecknika Inc., Austria). Murine macrophage cell line J774, obtained from ATCC, was cultured in Dulbecco's modified Eagle's medium (D-MEM, Gibco-BRL) supplemented with 10% FBS. Human peripheral blood monocytes were isolated from heparinized venous blood of normal donors by the Canadian Red Cross. Cells were centrifuged over Ficoll-Paque gradient (Pharmacia) as previously described (ref. 25). After several washes, cells were resuspended in RPMI 1640 medium (Gibco-BRL) containing 10% of human serum (Gibco-BRL). In order to differentiate monocytes into macrophages, 3 x 10⁶ peripheral blood leukocytes were adhered and cultured for 5 days at 37°C in 5% CO₂/95% air in a humidified atmosphere.

### Example 3

This Example describes *in vitro* macrophage infection.

The capacity of *Leishmania*-GM CSF transfectants to infect murine and human macrophages *in vitro* was tested in comparison to control *Leishmania-*pneo as follows. Murine and human macrophages were seeded (200 µl per well, 5 x 10⁴ cells/ml) into 8 wells chamber slides, and were infected with *L. major*-pneo and *L. major-hGM* CSF at a parasite to cell ratio of 20:1 for a period of 6 hours. The experiment was repeated with L. *donovani*-pneo and *L. donovani*-hGM CSF and -mGM CSF. Following this incubation, non-engulfed parasites were removed by 3 to 5 washes with warm medium and chambers were replenished with 500 µl of fresh culture medium. The level of infection was determined at 6, 24, 48, and 72 hours by optical microscopy examination following Diff Quick staining of cell preparations. The results obtained are shown in Figures 3 and 4 for *L. donovani* and *L. major* respectively. The results show that the GM CSF expression greatly reduces the viability of amastigotes inside murine or human macrophages.

### Example 4

This Example describes the neutralization of GM-CSF activity.

Two hundred µl of J774 murine macrophages were incubated for 1 hour at 37°C in the presence of 1 µg/ml of an anti-murine GM-CSF polyclonal antibody (R&D

Systems) prior to infection with *Leishmania* cells. Pre-incubated macrophages were then infected with *L major*-mGM CSF and wild-type parasites as described above in Example 3. The results obtained are shown in Figure 5. The anti-Leishmanial effect of GM-CSF was formed to be significantly decreased by the antibody treatment.

### Example 5

This Example describes ELISA immunoassays.

Supernatants from *L. major-* and *L. donovani-hGM* CSF-containing parasites and from controls carrying only the *neo* vector were harvested by centrifugation following 5 days of culture to stationary phase with a reached density of 2 x 10⁷ promastigotes/ml. Supernatants were assayed directly using a mAb ELISA kit (R&D Systems) as recommended by the manufacturers. Standard curves for quantification and comparison were generated using a recombinant hGM-CSF protein. *Leishmania* expressing the hGM-CSF gene were associated with detectable amounts of GM-CSF protein at the media, ranging from 2.8 to 6.2 ng/ml.

### SUMMARY OF THE DISCLOSURE

In summary of the present disclosure, there is provided a macrophage-infecting parasite expressing a granulocyte macrophage stimulating factor (GM-CSF) gene for use as a vaccine against or treatment of a parasitic infection. In particular, the parasite may be a strain of *Leishmania.*

### REFERENCES

1. WHO, Tropical Disease Report, 1989. pp. 85-92.
2. Turco, S.J. and Descoteaux, A., 1992. The lipophosphoglycan of *Leishmania* parasites. Annu. Rev. Microbiol. 46:65-94.
3. Modabber, F. 1989. Experiences with vaccines against cutaneous leishmaniasis: of men and mice. Parasitol. 98:349-360.
4. Coffman, R. et al. (1991) Immunol. Rev. **123:**190-207.
5. Locksley, R.M. and Scott, P. (1991). Immunol. Today 12A: 58-A61.
6. Doherty, T.M and Coffman, R.L (1993) J. Immunol. 150: 5476-5483.
7. Oster, C.N. and Nacy, C.A. (1984) J. Immunol. **132**: 1494.
8. Weiser, W.Y. et al. (1987) J. Exp. Med. **166:** 1436-1446.
9. Hart, P.H. et al. (1988) J. Immunol. **141:** 1516-1521.
10. Ho, J., Reed, S., Wick, E., and Giordano, M. (1990) J. Infect. Dis. **162**: 224-230.
11. Murray, H.W. (1994a) Bailliere's Clin. Infect. Dis. **1**: 237-246.
12. Titus, R.G., Sherry, B. and Cerami, A. (1989) J. Exp. Med. **170:** 2097-2104.
13. Liew, F.Y. et al. (1990a) Immunology **69:**570.
14. Heinzel, F.P. et al. (1993) J. Exp. Med. **177**: 1505-1509.
15. Tumang, M., Keogh, C., Moldawer, L., Helfgott, D., Hariprashad, J. and Murray, H. (1994) J. Immunol. **153**: 768-775.
16. Murray, H.W. and Hariprashad, J. (1995) J. Exp. Med. **181**: 387-391.
17. Murray, H.W. et al. (1995). J. Clin. Invest. **95**: 1183-1192.
18. Silverstein, D.S. et al. (1986) J. Immunol. **137:** 3290-3294.
19. Grabstein, K.H. et al. (1986) Science **232:** 506-508.
20. Reed, S.G. et al. (1987) J. Exp. Med. **166:** 1734-746.
21. Laban, A. et al. (1990) Nature **343:** 572.
22. Brun, R. and Schonenberger, M. (1979) Acta Trop. **36:** 289.
23 . Green, S.J. et al. (1990) J. Immunol. **145:**4290.
24. Muyombwe, A. et al. (1996) Exp. Parasitol. Submitted.
25. Olivier, M. et al.. (1992). J. Immunol. **148:** 1188-1196.
26. Bernards, A. et al. (1981). Cell, **27:** 497-505.
27. Murray, H.W. (1994b) Parasitol. Today **10**(6): 220-223.
28. Cervia, J. et al. (1993) Clin. Res. **41:** 337.
29. Handman, E., and Burgess, A.W. (1979). J. Immunol. **122**: 1134-1137.
30. Corcoran, L.M. et al. (1988) J. Parasitol. **74:** 763-767.
31. Greil, J. et al. (1988) Eur. *J.* Immunol. **18:** 1527-1537.
32. Liew, F.Y. et al. (1990b) *J.* Immunol. **145:**4306.

## Claims

1. A macrophage infecting parasitic protozoan of the order Kinetoplastidae expressing a granulocyte macrophage colony stimulating factor (GN-CSF) gene.

2. The parasitic protozoan claimed in claim 1 which is a strain of *Leishmania.*

3. The parasitic protozoan claimed in claim 2 wherein said strain of *Leishmania* is selected from the group consisting of *Leishmania donavani, Leishmania braziliensis, Leishmania tarentolae, Leishmania major, Leishmania mexicana, Leishmania tropica and Leishmania aethiopica.*

4. The parasitic protozoan claimed in any one of claims 1 to 3 which is reduced in the ability of said strain to infect or survive within macrophages.

5. The parasitic protozoan claimed in any one of claims 1 to 4, in which said GM-CSF gene is of murine origin.

6. The parasitic protozoan claimed in any one of claims 1 to 4, in which said GM-CSF gene is of human origin.

7. The parasitic protozoan claimed in any one of claims 1 to 6, in which said GM-CSF gene is expressed using the α-tubulin intergenic sequences of *Leishmania enrietti.*

8. The parasitic protozoan claimed in any one of claims 1 to 7, in which said GM-CSF gene is expressed from a plasmid.

9. The parasitic protozoan claimed in any one of claims 1 to 8, in which at least one gene of the parasitic protozoan contributing to virulence thereof has been functionally disabled.

10. The parasitic protozoan claimed in any one of claims 1 to 9 which expresses at least one additional cytokine.

11. An immunogenic composition comprising an attenuated form of the parasitic protozoan claimed in any one of claims 1 to 10.

12. The immunogenic composition claimed in claim 11, in which the parasitic protozoan is a strain of *Leishmania* and the composition is formulated for *in vivo* administration to a host infected by *Leishmania* to treat said infection.

13. The immunogenic composition claimed in claim 11, in which said parasitic protozoan is a strain of *Leishmania* and said composition is formulated as a vaccine for *in vivo* administration to a host to confer protection against disease caused by a virulent strain of *Leishmania.*

14. The immunogenic composition claimed in claim 12 or 13, in which the host is a primate.

15. The immunogenic composition claimed in claim 12 or 13 in which the host is a human.

16. A parasitic protozoan as claimed in any one of claims 1 to 11 for use as a medicament.

17. The use of a parasitic protozoan as claimed in any one of claims 1 to 11 in the manufacture of an immunogenic composition.

18. The use of claim 17, in which said parasitic protozoan is a strain of *Leishmania* and said immunogenic composition is formulated for *in vivo* administration to a host infected with *Leishmania* to treat said infection.

19. The use of claim 17, in which said parasitic protozoan is a strain of *Leishmania* and said composition is formulated as a vaccine for *in vivo* administration to a host to confer protection against disease caused by a virulent strain of *Leishmania.*

20. The use of claim 19, in which the virulent strain is selected from the group consisting of *Leishmania donovani, Leishmania major, Leishmania mexicana, Leishmania tropica* and *Leishmania aethiopica.*

## Patentansprüche

1. Ein Makrophagen infizierender parasitischer Protozoe der Ordnung Kinetoplastidae, welcher ein Gen des Granulozyten Makrophaaen koloniestimulierenden Faktors (GM-CSF) exprimiert.

2. Der in Anspruch 1 beanspruchte parasitische Protozoe, welcher ein *Leishmania*-Stamm ist.

3. Der in Anspruch 2 beanspruchte parasitische Protozoe, wobei der *Leishmania-*Stamm ausgewählt ist aus der Gruppe, bestehend aus *Leishmania donavani, Leishmania brasiliensis, Leish*mania *tarentolae, Leishmania major, Leishmania mexicana, Leishmania tropica und Leishmania aethiopica.*

4. Der in irgendeinem der Ansprüche 1 bis 3 beanspruchte parasitische Protozoe, welcher im Hinblick auf die Fähigkeit des Stammes, Makrophagen zu infizieren oder innerhalb dieser zu überleben, eingeschränkt ist.

5. Der in irgendeinem der Ansprüche 1 bis 4 beanspruchte parasitische Protozoe, in welchem das GM-CSF-Gen aus Mäusen stammt.

6. Der in irgendeinem der Ansprüche 1 bis 4 beanspruchte parasitische Protozoe, in welchem das GM-CSF-Gen aus dem Menschen stammt.

7. Der in irgendeinem der Ansprüche 1 bis 6 beanspruchte parasitische Procozoe, in welchem das GM-CSF-Gen unter Verwendung der intergenen α-Tubulinsequenzen von Leishmania enrietti exprimiert wird.

8. Der in irgendeinem der Ansprüche 1 bis 7 beanspruchte parasitische Protozoe, in welchem das GM-CSF-Gen von einem Plasmid exprimiert wird.

9. Der in irgendeinem der Ansprüche 1 bis 8 beanspruchte parasitische Protozoe, in welchem wenigstens ein Gen des parasitischen Protozoen, das zu dessen Virulenz beiträgt, in seiner Funktion gestört wurde.

10. Der in irgendeinem der Ansprüche 1 bis 9 beanspruchte parasitische Protozoe, welcher wenigstens ein zusätzliches Zytokin exprimiert.

11. Eine immunogene Zusammensetzung, welche eine abgeschwächte Form des in irgendeinem der Ansprüche 1 bis 10 beanspruchten parasitischen Protozoen umfaßt.

12. Die in Anspruch 11 beanspruchte immunogene Zusammensetzung, in welcher der parasitische Protozoe ein *Leishmania*-Stamm ist und die Zusammensetzung zur *in vivo*-Verabreichung an einen Wirt, der mit *Leishmania* infiziert ist, formuliert ist, um diese Infektion zu behandeln.

13. Die in Anspruch 11 beanspruchte immunogene Zusammensetzung, in welcher der parasitische Protozoe ein *Leishmania*-Stamm ist und die Zusammensetzung als ein Impfstoff für eine *in vivo*-Verabreichung an einen Wirt formuliert ist, um einen Schutz gegenüber einer Krankheit zu verleihen, die durch einen virulenten *Leishmania*-Stamm verursacht wird.

14. Die in Anspruch 12 oder 13 beanspruchte immunogene Zusammensetzung, wobei der Wirt ein Primat ist.

15. Die in Anspruch 12 oder 13 beanspruchte immunogene Zusammensetzung, wobei der Wirt ein Mensch ist.

16. Ein parasitischer Protozoe, wie er in irgendeinem der Ansprüche 1 bis 11 beansprucht wird, zur Verwendung als ein Medikament.

17. Die Verwendung eines parasitischen Protozoen, wie er in irgendeinem der Ansprüche 1 bis 11 beansprucht wird, bei der Herstellung einer immunogenen Zusammensetzung.

18. Die Verwendung nach Anspruch 17, bei welcher der parasitische Protozoe ein *Leishmania*-Stamm ist und die immunogene Zusammensetzung zur *in vivo*-Verabreichung an einen Wirt, welcher mit *Leishmania* infiziert ist, formuliert ist, um die Infektion zu behandeln.

19. Die Verwendung nach Anspruch 17, bei welcher der parasitische Protozoe ein *Leishmania*-Stamm ist und die Zusammensetzung als ein Impfstoff zur in vivo-Verabreichung an einen Wirt formuliert ist, um einen Schutz gegenüber einer Krankheit zu verleihen, die von einem virulenten *Leishmania*-Stamm verursacht wird.

20. Die Verwendung nach Anspruch 19, bei welcher der virulente Stamm ausgewählt ist aus der Gruppe, bestehend aus *Leishmania donovani, Leishmania major, Leishmania mexicana,* Leishmania *tropica und Leishmania aethiopica.*

## Revendications

1. Protozoaire parasitaire infectant les macrophages, de l'ordre des Kinetoplastidae, exprimant un gène de facteur stimulant les colonies de granulocytes et de macrophages (GN-CSF).

2. Protozoaire parasitaire selon la revendication 1, qui est une souche de *Leishmania.*

3. Protozoaire parasitaire selon la revendication 2, où ladite souche de *Leishmania* est choisie dans le groupe consistant en *Leishmania donavani, Leishmania braziliensis, Leishmania tarentolae, Leishmania major, Leishmania mexicana, Leishmania tropica* et *Leishmania aethiopica.*

4. Protozoaire parasitaire selon l'une quelconque des revendications 1 à 3, dont l'aptitude de ladite souche à infecter ou survivre dans les macrophages est réduite.

5. Protozoaire parasitaire selon l'une quelconque des revendications 1 à 4, dans lequel ledit gène de GM-CSF est d'origine murine.

6. Protozoaire parasitaire selon l'une quelconque des revendications 1 à 4, dans lequel ledit gène de GM-CSF est d'origine humaine.

7. Protozoaire parasitaire selon l'une quelconque des revendications 1 à 6, dans lequel ledit gène de GM-CSF est exprimé au moyen des séquences intergéniques d'α-tubuline de *Leishmania enrietti.*

8. Protozoaire parasitaire selon l'une quelconque des revendications 1 à 7, dans lequel ledit gène de GM-CSF est exprimé à partir d'un plasmide.

9. Protozoaire parasitaire selon l'une quelconque des revendications 1 à 8, dans lequel au moins un gène du protozoaire parasitaire contribuant à sa virulence a été désactivé fonctionnellement.

10. Protozoaire parasitaire selon l'une quelconque des revendications 1 à 9, qui exprime au moins une cytokine supplémentaire.

11. Composition immunogène comprenant une forme atténuée du protozoaire parasitaire selon l'une quelconque des revendications 1 à 10.

12. Composition immunogène selon la revendication 11, dans laquelle le protozoaire parasitaire est une souche de *Leishmania* et la composition est formulée pour l'administration in vivo à un hôte infecté par *Leishmania* pour traiter ladite infection.

13. Composition immunogène selon la revendication 11, dans laquelle ledit protozoaire parasitaire est une souche de *Leishmania* et ladite composition est formulée sous forme d'un vaccin pour l'administration in vivo à un hôte pour conférer une protection contre une maladie causée par une souche virulente de *Leishmania.*

14. Composition immunogène selon la revendication 12 ou 13, dans laquelle l'hôte est un primate.

15. Composition immunogène selon la revendication 12 ou 13, dans laquelle l'hôte est un humain.

16. Protozoaire parasitaire selon l'une quelconque des revendications 1 à 11 destiné à être utilisé comme médicament.

17. Utilisation d'un protozoaire parasitaire selon l'une quelconque des revendications 1 à 11 dans la production d'une composition immunogène.

18. Utilisation selon la revendication 17, dans laquelle ledit protozoaire parasitaire est une souche de *Leishmania* et ladite composition immunogène est formulée pour l'administration in vivo à un hôte infecté par *Leishmania* pour traiter ladite infection.

19. Utilisation selon la revendication 17, dans laquelle ledit protozoaire parasitaire est une souche de *Leishmania* et ladite composition est formulée sous forme d'un vaccin pour l'administration in vivo à un hôte pour conférer une protection contre une maladie causée par une souche virulente de *Leishmania*.

20. Utilisation selon la revendication 19, dans laquelle la souche virulente est choisie dans le groupe consistant en *Leishmania donovani, Leishmania major, Leishmania mexicana, Leishmania tropica* et *Leishmania aethiopica.*
